# EUROPEAN PATENT SPECIFICATION

(11) **EP 1 272 048 B1**
(45) Date of publication and mention of the grant of the patent: **21.05.2008**
(21) Application number: 01925818.5
(22) Date of filing: 12.04.2001
(51) Int. Cl.: A23J 1/08, C11B 1/00, C11B 7/00, A23D 9/013, A23J 7/00, A23L 1/32

(54) **METHOD FOR THE FRACTIONATION OF OIL AND POLAR LIPID-CONTAINING NATIVE RAW MATERIALS**
VERFAHREN ZUR FRAKTIONIERUNG VON ÖL- UND POLAR LIPID-ENTHALTENDEN ROHSTOFFEN
PROCEDE DE FRACTIONNEMENT DE MATIERES PREMIERES NATURELLES CONTENANT DE L'HUILE ET DES LIPIDES POLAIRES

(30) Priority: 12.04.2000 DE 10018213; 23.02.2001 US 271209 P
(43) Date of publication of application: 08.01.2003
(73) Proprietor: Westfalia Separator GmbH, 59302 Oelde (DE)
(72) Inventor: HRUSCHKA, Steffen, M., 59302 Oelde (DE); KIRCHNER, Stefan, 33334 Gütersloh (DE); RASSENHOVEL, Jurgen, 59302 Oelde (DE); WITT, Willi, 49545 Tecklenburg (DE); GUSEK, TODD W., Crystal, Minnesota, USA 55422 (US); EFSTATHIOU, JOHN D., Plymouth, Minnesota, USA 55441 (US)
(74) Representative: Specht, Peter
(86) International application number: PCT/IB2001/000841
(87) International publication number: WO 2001/076715

(56) References cited:
- WO-A-93/22931
- CA-A- 1 335 054
- US-A- 4 357 353
- US-A- 5 780 095
- US-A- 5 883 273
- DATABASE FSTA [Online] INTERNATIONAL FOOD INFORMATION SERVICE (IFIS), FRANFURT/MAIN, DE; AN:80-3-03-q0050, XP002177927 & MILEWSKI J; RUTKOWSKI A: "Solubility of egg yolk lipids in an aqueous ethanol medium" ZESZYTY NAUKOWE SZKOLY GLOWNEJ GOSPODARSTWA WIEJSKIEGO AKADEMII, no. 12, 1978, pages 95-0102, Warsaw, Poland

## Description

### FIELD OF THE INVENTION

The present invention relates to the fields of extraction, separation and recovery, and in particular, the extraction, separation and recovery of polar lipid-rich fractions from mixtures such as native raw materials. Other fractions in the raw materials can be simultaneously recovered and these fractions, such as a protein-rich fraction, retain much or all of their original functionality because of the mild conditions utilized in the extraction process.

### BACKGROUND OF THE INVENTION

Examples of polar lipids include phospholipids (e.g. phosphatidyl choline, phosphatidyl ethanolamine, phosphatidyl inositol, phosphatidyl serine, phosphatidylglycerol, diphosphatidylglycerols), cephalins, sphingolipids (sphingomyelins and glycosphingolipids), and glycoglycerolipids. Phospholipids are composed of the following major structural units: fatty acids, glycerol, phosphoric acid, amino alcohols, and carbohydrates. They are generally considered to be structural lipids, playing important roles in the structure of the membranes of plants, microbes and animals. Because of their chemical structure, polar lipids exhibit a bipolar nature, exhibiting solubility or partial solubility in both polar and non-polar solvents. The term polar lipid within the present description is not limited to natural polar lipids but also includes chemically modified polar lipids. Although the term oil has various meanings, as used herein, it will refer to the triacylglycerol fraction.

One of the important characteristics of polar lipids, and especially phospholipids, is that they commonly contain polyunsaturated fatty acids (PUFAs: fatty acids with 2 or more unsaturated bonds). In many plant, microbial and animal systems, they are especially enriched in the highly unsaturated fatty acids (HUFAs: fatty acids with 4 or more unsaturated bonds) of the omega-3 and omega-6 series. Although these highly unsaturated fatty acids are considered unstable in triacylglycerol form, they exhibit enhanced stability when incorporated in phospholipids.

The primary sources of commercial PUFA-rich phospholipids are soybeans and canola seeds. These biomaterials do not contain any appreciable amounts of HUFAs unless they have been genetically modified. The phospholipids (commonly called lecithins) are routinely recovered from these oilseeds as a by-product of the vegetable oil extraction process. For example, in the production of soybean or canola oil, the beans (seeds) are first heat-treated and then crushed, ground, and/or flaked, followed by extraction with a non-polar solvent such as hexane. Hexane removes the triacylglycerol-rich fraction from the seeds together with a varying amount of polar lipids (lecithins). The extracted oil is then de-gummed (lecithin removal) either physically or chemically as a part of the normal oil refining process and the precipitated lecithins recovered. This process however has two disadvantages: (1) the seeds must be heat-treated before extraction with hexane, both increasing the processing cost and denaturing the protein fraction, thereby decreasing its value as a by-product; and (2) the use of the non-polar solvents such as hexane also presents toxicity and flammability problems that must be dealt with.

The crude lecithin extracted in the "de-gumming" process can contain up to about 33% oil (triacylglycerols). One preferred method for separating this oil from the crude lecithin is by extraction with acetone. The oil (triacylglycerols) is soluble in acetone and the lecithin is not. The acetone solution is separated from the precipitate (lecithin) by centrifugation and the precipitate dried under first a fluidized bed drier and then a vacuum drying oven to recover the residual acetone as the product is dried. Drying temperatures of 50-70°C are commonly used. The resulting dried lecithins contain approximately 2-4% by weight of oil (triacylglycerols). Process temperatures above 70°C can lead to thermal decomposition of the phospholipids. However, even at temperatures below 70°C the presence of acetone leads to the formation of products that can impair the organoleptic quality of the phospholipids. These by-products can impart musty odors to the product and also a pungent aftertaste.

To avoid use of non-polar solvents such as hexane and avoid the negative side effects of an acetone-based process, numerous processes have also been proposed involving the use of supercritical fluids, especially supercritical CO₂. For example, U.S. Patent No. 4,367,178 discloses the use of supercritical CO₂ to partially purify crude soy lecithin preparation by removing the oil from the preparation. German Patent Nos. DE-A 30 11 185 and DE-A 32 29 041 disclose methods for de-oiling crude lecithin with supercritical CO₂ and ethane respectively. Other supercritical processes have been proposed which include adding small amounts of hydrocarbons such as propane to the supercritical CO₂ to act as entraining agents. However, supercritical fluid extraction systems are very capital expensive and cannot be operated continuously. Further, extraction times are long and the biomaterials must be dried before extraction, and this increases the difficulties of stabilizing the resulting dry product with antioxidants. All of these factors make the supercritical process one of the most expensive options for extracting and recovering polar-lipid material or mixtures of these materials. As a result, alternative processes using extraction with liquid hydrocarbons at lower pressures have been described. For example U.S. Patent No. 2,548,434 describes a method for de-oiling oilseed materials and recovering crude lecithin using a liquid hydrocarbon at lower pressures (35-45 bars) but elevated temperatures (79° to 93°C). U.S. Patent No. 5,597,602 describes a similar process that operates at even lower pressures and temperatures. However, even with these improvements supercritical fluid extraction remains very expensive and is not currently used to produce phospholipids for food use on a large commercial scale.

The primary commercial source of HUFA-rich polar lipids is egg yolk. Two primary methods are used for the recovery of egg phospholipids on an industrial scale. Both require the drying of the egg yolk before extraction. In the first process the dried egg yolk powder is extracted first with acetone to remove the triacylglycerols. This is then followed by an extraction with pure alcohol to recover the phospholipids. In the second process, pure alcohol is used to extract an oil/lecithin fraction from the dried egg yolk. The oil/lecithin phase is then extracted with acetone to remove the triacylglycerols, leaving behind a lecithin fraction. There are several disadvantages to both of these methods: (1) the egg yolk must first be dried before processing, an expensive step, and additionally this drying process can damage and denature the proteins, severely reducing their value as a food ingredient; (2) the alcohol and acetone concentrations used in these processes must be above 80%, and preferably higher than 90% in concentration, to be effective. Higher purity solvents are more expensive and use of high solvent concentrations leads to denaturation of the proteins, reducing their value; and (3) separate solvent recovery conditions must be available to recover two types of solvents, increasing the cost of equipment. All three of these disadvantages lead to significant increases in the costs of separating and recovering polar lipid-rich fractions from egg yolk.

Canadian Patent No. 1,335,054 describes a process for extracting fresh liquid egg yolk into protein, oil and lecithin fractions by the use of ethanol, elevated temperatures, filtration and low temperature crystallization. The process however has several disadvantages: (1) denaturation of the protein due to the use of high concentrations of ethanol; (2) the process is limited to ethanol; (3) the process removes the protein first and then the lecithins are recovered from the oil fraction. The purity of the lecithin product is not disclosed.

US-A-5 883 273 (MCCOMBS CHARLES ALLAN ET AL) discloses a process comprising extracting lipids from egg yolk with a lower alkyl alcohol, in particular from egg yolk solids with methanol. Furthermore, water can be used to assist in such separation process and the quantity of water can vary over a range of from about 1 wt% to about 100 wt% based on the source of lipids, e.g., egg yolk solids.

DATABASE FSTA (Online) INTERNATIONA FOOD INFORMATION SERVICE (IFIS), Frankfurt/Main, DE; AN:80-3-03-q0050, XP002177927 & Milewski J; Rutkowski A: "Solubility of egg yolk lipids in an aqueous ethanol medium" Zeszyty Naukowe Szkoly Glownej Gospodarstwa Wiejskiego Akademii, Nr. 12, 1978, pages 95-0102, Warsaw, Poland, discloses the extraction of lipids from egg yolk at room temperature by using water/ethanol mixtures containing 0, 2550, 75 or 96 vol.% ethanol.

US-A-4 357 353 (Strauss Kuno et at) discloses a solvent extraction of a specific phospholipid mixture, e.g. egg lecithin by using alcohol with sufficient water, in particular 65 to 96 % based on the resulting solvent mixture to cause the partial precipitation of undissolved components of the phospholipids mixture whereafter the said components are separated and dried.

In light of the current state of the art, there remains a need for an improved extraction technology for food-grade polar lipid products which is less expensive to operate, which protects the value of the associated by-products, and which protects the overall quality of the HUFAs in the polar lipid products.

### SUMMARY OF THE INVENTION

In accordance with the present invention, an improved process is provided for recovering polar lipids from native biomaterials, which does not involve all of the disadvantages of the prior art. The invention resides in a process for recovering polar lipids and/or polar lipid-containing mixtures from partially or completely de-oiled biomaterials using considerably lower alcohol concentrations than hitherto thought possible. The invention also provides an improved process for de-oiling the biomaterials prior to extraction/recovery by the methods outlined in the invention.

In accordance with the present invention, processes are provided for fractionation of a low-oil content, polar lipid-containing material according to claim 8, 10 and 11. The process includes the steps of blending the low-oil content, polar lipid-containing material with water and a water-soluble organic solvent and subjecting the mixture to density separation (e.g., using gravity or centrifugal forces) to separate it into a light phase and a heavy phase. Preferably the light phase comprises a polar lipid-rich fraction and the heavy phase comprised a protein-rich fraction. "Low-oil content" means that the polar lipid-containing material has less than 20% dry weight of triacylglycerols, preferably less than 15%, more preferably less than 10% and most preferably less than 5%. The low-oil content, polar lipid-rich material can be obtained by removing oil from a polar lipid-rich material or by selecting a polar lipid-rich material with a low oil content. For example, some plant materials (other than oilseeds) and some microbes can be used as polar lipid-rich materials having a low oil content. Preferably, at least 60% and more preferably at least 80% of the polar lipids originally present in the low-oil content, polar lipid-containing material are recovered in a polar lipid-rich light phase.

In accordance with another embodiment of the present invention, a process for fractionation of an oil-, polar lipid-, and protein-containing mixture is provided. The process includes the steps of separating oil from the mixture to form an oil-rich fraction and a polar lipid/protein-rich fraction, adding water-soluble organic solvent to the polar lipid/protein-rich fraction, and subjecting the water-soluble organic solvent and polar lipid/protein-rich fraction to density separation, eg. using gravity or centrifugal force, to form a polar lipid-rich fraction and a protein-rich fraction. Preferably, at least 60% and more preferably at least 80% of the polar lipids originally present in the mixture are recovered in a polar lipid-rich fraction.

In accordance with another embodiment of the present invention, a process for recovering polar lipid from a polar lipid-containing mixture employing the use of a water-soluble organic solvent, wherein the relatively high solubility of polar lipid in an aqueous solution of the water-soluble organic solvent, in which the water-soluble organic solvent comprises less than 35 percent by weight or more than 68 percent by weight of the aqueous solution, is employed to assist in the recovery.

In accordance with another embodiment of the present invention, a process for fractionation of an oil-, polar lipid-, and protein-containing mixture is provided. The process includes the steps of adding water-soluble organic solvent to the oil-, polar lipid-, and protein-containing mixture, subjecting the water-soluble organic solvent and oil-, polar lipid-, and protein-containing mixture to homogenisation, and separating oil from the mixture to form an oil-rich fraction and a polar lipid/protein-rich fraction.

In accordance with another embodiment of the present invention there is provided a process for fractionation of an oil-, polar lipid-, and protein-containing mixture. The process comprises the steps of separating oil from said mixture to form an oil-rich fraction and a polar lipid/protein-rich fraction, adding water-soluble organic solvent to said polar lipid/protein-rich fraction, and subjecting the water-soluble organic solvent and polar lipid/protein-rich fraction to density separation to form a polar lipid-rich fraction and a protein-rich fraction. The separation of oil fro the mixture comprises the steps of homogenisation of said oil-, polar lipid-, and protein-containing mixture, adding water-soluble organic solvent and water to said mixture, and separating the resulting mixture into an oil-rich fraction and a polar lipid/protein-rich fraction.

In accordance with another embodiment of the present invention there is provided a process for fractionation of an oil-, polar lipid-, and protein-containing mixture. The process comprises the steps of separating oil from said mixture to form an oil-rich fraction and a polar lipid/protein-rich fraction, adding water-soluble organic solvent to said polar lipid/protein-rich fraction, and subjecting the water-soluble organic solvent and polar lipid/protein-rich fraction to density separation to form a polar lipid-rich fraction and a protein-rich fraction. The separation of oil from the mixture comprises the steps of adding water-soluble organic solvent and water to said mixture, homogenisation of said oil-,polar lipid-, and protein-containing mixture, and separating the resulting mixture into an oil-rich fraction and a polar lipid/protein-rich fraction.

An advantage of the present invention, at least according to one embodiment, is that it is significantly less costly than other known methods. An advantage of the present invention, at least according to one embodiment, is that it protects other by-products such as extracted protein from degradation increasing their value as by-products for sale. An advantage of the present invention, at least according to one embodiment, is that it protects the HUFAs in the polar lipids from degradation. These advantages result from some of the key aspects of the invention: (1) the biomaterials do not need to be dried prior to de-oiling; (2) the process uses low concentrations of alcohol; (3) the quality and functionality of associated by-products are protected from degradation (eg. denaturation of proteins by high temperatures or high solvent concentrations; oxidation of lipids; formation of unwanted by-products); and (4) the overall process is very simple (both in terms of equipment and processing steps). Preferably, the process steps are conducted under oxygen-reduced atmospheres that can include use of inert or non-reactive gases (e.g. nitrogen, carbon dioxide, argon, etc), use of solvent vapors, use of a partial or full vacuum, or any combination of the above.

### BRIEF DESCRIPTION OF THE FIGURES

The present invention may be more readily understood by reference to the following figures, wherein
FIG. 1 is a graphical representation of the solubility of phospholipids, a form of polar lipids, as a function of alcohol concentration;
FIG. 2 is a graphical representation of the effect of homogenization on the de-oiling of egg yolk;
FIG. 3 is a graphical representation of a phospholipid extraction process (as an example of a polar lipid extraction process) based on a low concentration of alcohol;

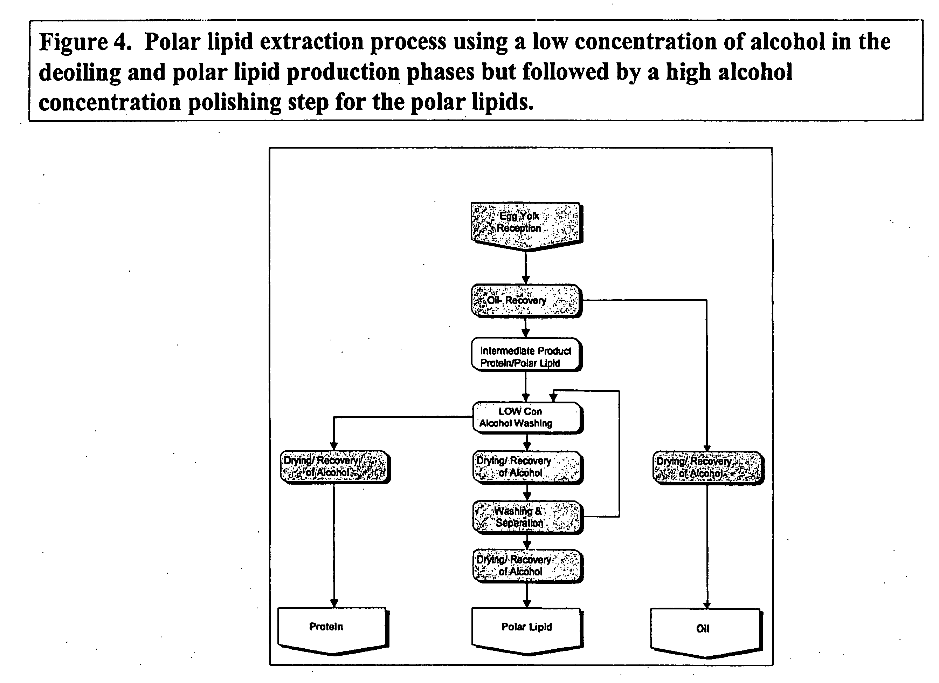

FIG. 4 is a graphical representation of a phospholipid extraction process (as an example of a polar lipid extraction process) based on a low concentration of alcohol but with the additional step of polishing the phospholipids with a step utilizing a high concentration of alcohol; and

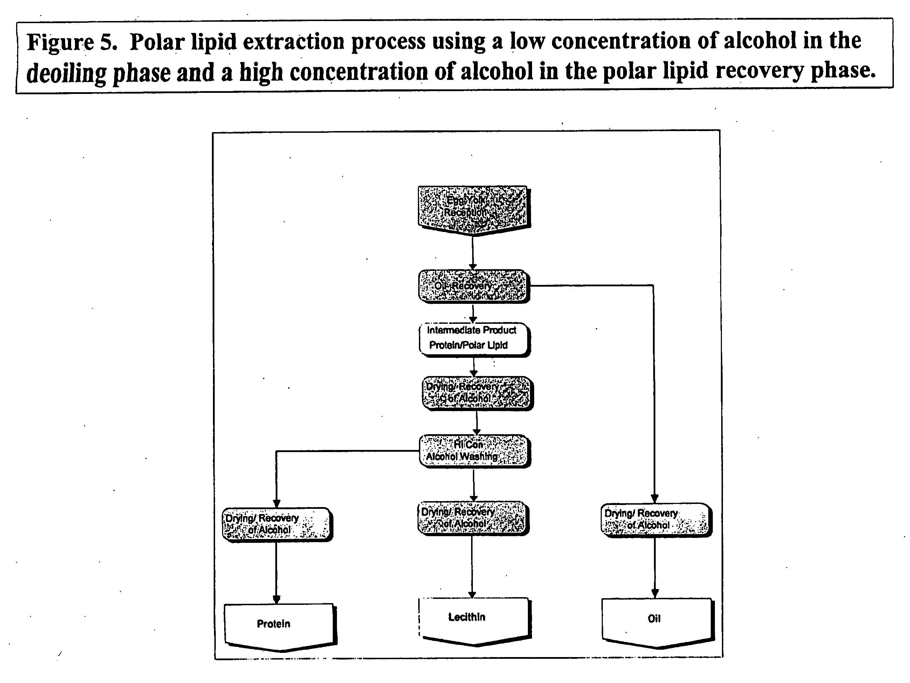

FIG. 5 is a graphical representation of a phospholipid extraction (as an example of a polar lipid extraction process) process based on using a high concentration of alcohol throughout the phospholipid recovery portion of the extraction process.

### DETAILED DESCRIPTION OF THE INVENTION

Because of their bipolar nature, polar lipids (including phospholipids) are of significant commercial interest as wetting and emulsifying agents. These properties may also help make HUFAs in the phospholipids more bioavailable, in addition to enhancing their stability. These properties make phospholipids ideal forms of ingredients for use in nutritional supplements, food, infant formula and pharmaceutical applications.

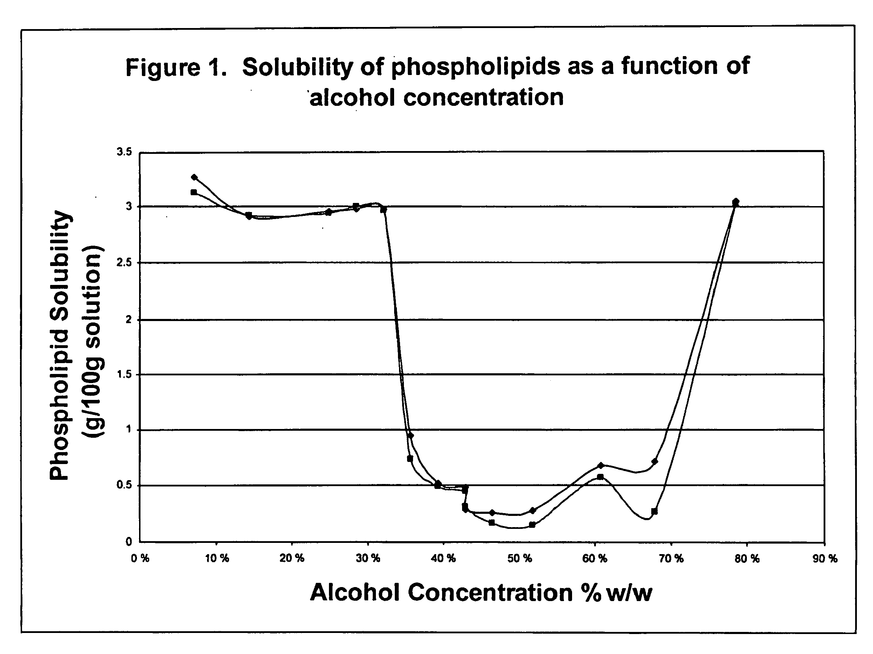

We have unexpectedly found that polar lipids are very soluble not only in high alcohol concentrations (e.g. at alcohol concentrations greater than 68%) but also in low alcohol concentrations (less than 35% alcohol) (FIG. 1). For the purpose of this invention, phospholipids are described as "soluble" if they do not settle or separate from the continuous phase (sometimes also called supernatant or light phase) when subjected to centrifugation by the types of equipment described in this application. In the alcohol concentration range from 35% w/w to 68% w/w alcohol, polar lipids exhibit significantly lower solubility. The present invention exploits this property of polar lipids (enhanced solubility/dispersibility at low alcohol concentrations) which can then be exploited in several ways to develop processes for inexpensively extracting and recovering polar lipids, and especially phospholipids, from native biomaterials.

Native biomaterials that are rich in HUFA-containing polar lipids include fish, crustaceans, microbes, eggs, brain tissue, milk, meat and plant material including oilseeds. As used herein, the terms fish, crustaceans, microbes, eggs, brain tissue, milk, meat and plant material including oilseeds will include genetically modified versions thereof. The content of phospholipids in these materials is generally low, usually ranging from 0.1% to 4% by wet weight. As a result large amounts of raw materials need to be processed to recover these phospholipids. Because of the high costs of prior extraction techniques, phospholipids and especially HUFA-enriched phospholipids were very expensive and therefore restricted to use in the infant formula, pharmaceutical and cosmetic industries. One of the advantages of the present invention is that it provides for the extraction of polar lipids, and in particular phospholipids, in a cost-effective manner.

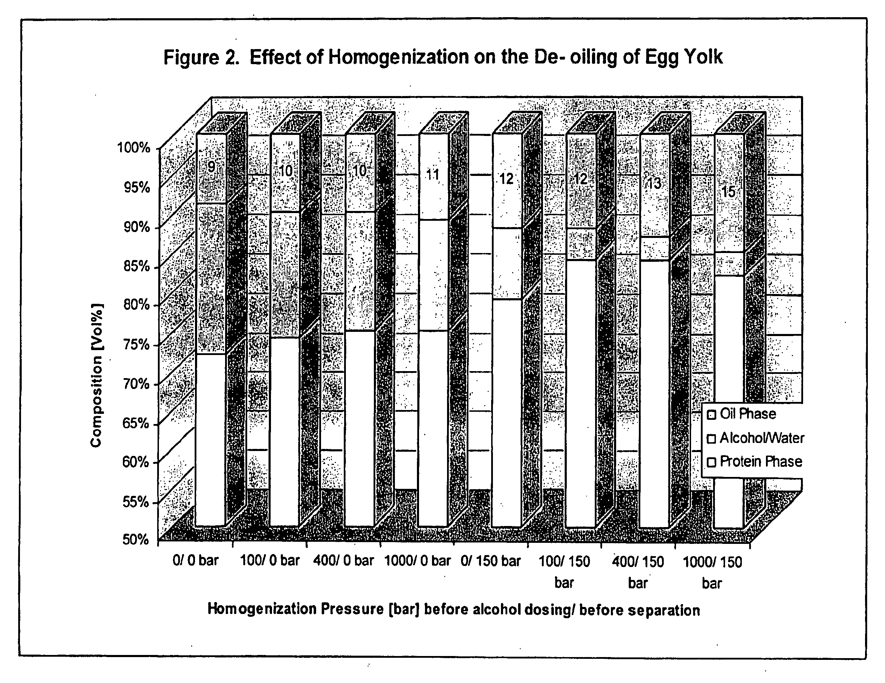

In the first step of one embodiment of the process of the present invention, a low-oil content material is selected or the material is de-oiled by any suitable de-oiling process, but preferably by a de-oiling process that does not cause denaturation of the proteins. This would include processes that do not utilize high temperatures (e.g. greater than 65°C) or high concentrations of solvents (e.g. greater than 50%). Preferably the de-oiling process outlined in WO 96/05278 (U.S. Patent No. 5,928,696) is utilized. Preferably, a key change is made to this de-oiling process. We have unexpectedly found that homogenizing the biomaterial prior to addition of the alcohol and water, or homogenization after the addition of the alcohol and water, but most preferably homogenization both prior to and after addition of alcohol and water, leads to improvements in oil recovery up to 85% higher than without homogenization (FIG. 2). As used herein, homogenization can include any high shear process such as processing the mixture under pressure through a small orifice, using a colloidal mill, or other high shear process, etc. Preferably, when the mixture is forced through a small orifice, the homogenization is conducted at pressures from 100 bars to 1000 bars, and more preferably from 150 bars to 350 bars. This is an unexpected result, as one skilled in the art would expect that homogenization of this type of mixture would lead to formation of very strong emulsions which would be very difficult to break, making the process less efficient.

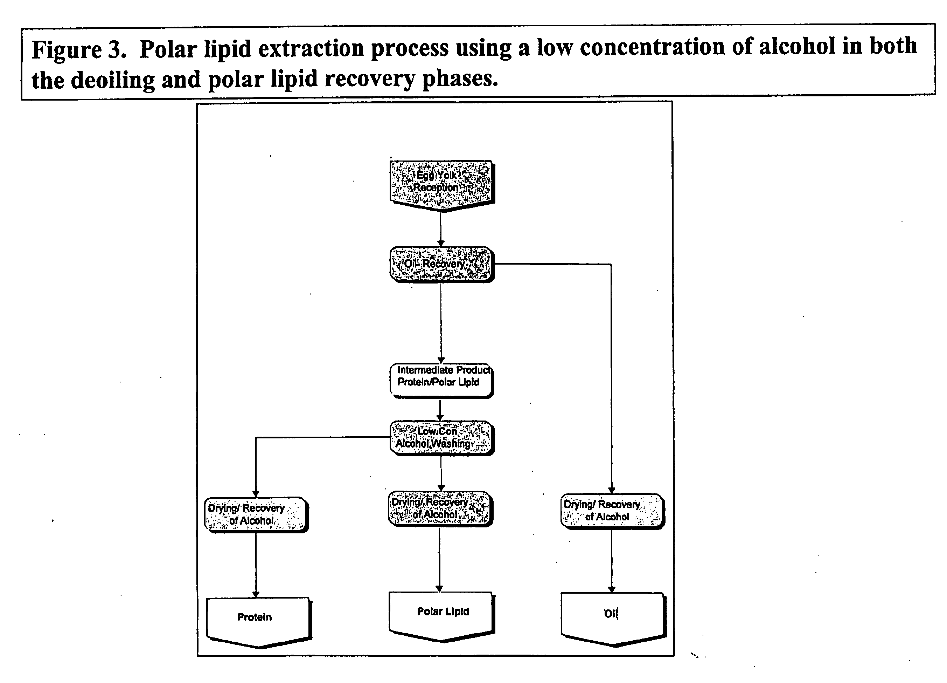

A lecithin recovery process utilizing low concentrations of alcohol throughout the entire process is outlined in FIG. 3. Liquid egg yolk is used as the polar-lipid rich biomaterial in this example. It is understood, however, that other polar lipid-containing biomaterials (e.g. fish, crustaceans, microbes, brain tissue, milk, meat and plant material including oilseeds) could also be processed in a similar manner with minor modifications to the process. In the first step of the process, the material is de-oiled by any well-known de-oiling process, but preferably by a de-oiling process that does not cause denaturation of the proteins. For a more efficient recovery of the oil, the material is sheared by means of homogenization to break up the fat-containing cellular particles so that the oil in the particles can be separated as well as the free oil in the biomaterial. Alcohol and water are then added to the yolk and the mixture is re-homogenized. The concentration of alcohol in the aqueous solution can be from 5 to 35% w/w, preferably from 20 to 35% w/w, and most preferably from 25 to 30% w/w. The free oil is then separated by means of centrifugal force due to a difference in density. This results in two fractions being recovered: (1) a fraction with approximately 50-70% protein (as % dry weight) and 30-50% dry weight as polar lipids, the mixture containing a significantly lower cholesterol content that the egg yolk; and (2) an egg oil with approximately 85% of the triacylglycerols of the egg yolk. Additional dosing of the protein/lecithin fraction with low concentration alcohol disperses the lecithin that is then separated from the protein by means of centrifugal force. Counter-current washing/centrifugation or cross-current washing/separation of the protein and lecithin products can be employed to improve the purity of the products and economics of the overall process. The protein is not denatured in this process and retains high resale value (because of its functionality) as a by-product of the process thereby reducing overall costs of all products produced.

Because of the simplicity of the equipment required in the process, the entire process can very easily be conducted under a reduced-oxygen atmosphere (e.g., nitrogen, a preferred embodiment of the process), further protecting any HUFAs in the polar lipids from oxidation. For example, a gas tight decanter can be used to separate oil from the mixture. A suitable decanter is model CA 226-28 Gas Tight available from Westfalia Separator Industry GmbH of Oelde Germany, which is capable of continuous separation of oil from suspensions with high solids content in a centrifugal field. A gas tight separator useful for separating polar lipids from proteins is model SC 6-06-576 Gas Tight available from Westfalia Separator Industry GmbH of Oelde Germany, which is capable of continuous separation of solids from suspensions with high solids content in a centrifugal field.

An improved version of this process has also been developed. In this process the de-oiling and lecithin washing steps employing low alcohol concentrations are similar to the process outlined above. However after the lecithin phase is dried, it is washed with concentrated alcohol. Since proteins are not soluble in high concentrations of alcohol, they precipitate (while the lecithin dissolves) and the precipitated proteins are separated by density separation, e.g., using gravity or centrifugal force. The protein-reduced lecithin is then concentrated by means of evaporation of water and alcohol. The advantage of this variation of the process is that it provides options for the production of both higher and lower quality lecithin fractions, and in providing the higher quality lecithin, only a very small portion of the protein is denatured.

The process has also been modified for use of high concentrations of alcohol after the de-oiling step. The process steps after de-oiling the biomaterials are similar to the low alcohol concentration process, but instead of diluted alcohol, concentrated alcohol is added. After de-oiling, concentration and drying of the polar lipid/protein intermediate product takes place. The concentration/drying step is necessary to reduce the amount of concentrated alcohol necessary to be added to re-dissolve the polar lipids. The dried polar lipid/protein phase is washed with concentrated alcohol and the protein precipitates. The precipitated protein is separated by density separation, e.g., using gravity or centrifugal force, in a counter-current washing system. The protein-reduced polar lipids are concentrated by means of evaporation of alcohol and water. The advantage of this process is that it requires lower thermal energy inputs: The major disadvantage is that all of the protein is denatured and is of lower value.

While not wishing to be bound by any theory, it is believed that several of the underlying mechanisms in the processes above are as discussed in further detail below. With regard to homogenization it is believed that destruction of cellular material occurs here. An objective is to achieve homogeneous distribution of all components, i.e., to create a homogeneous polydisperse system (protein, oil, lipoproteins, continuous phase water), so that, when aqueous or pure alcohol is added, this can immediately be uniformly, i.e., homogeneously, distributed without causing local irreversible protein denaturation. The temperature is to be kept as low as possible, so that as little lecithin as possible is dissolved in the oil phase. The pressure employed in the homogenization process should preferably be less than 1000 bars, and more preferably less than 600 bars, in order to destroy the quaternary and tertiary structure of the proteins, but not the primary and secondary structure. The concentration of alcohol is preferably less than 30% w/w, more preferably 28%. An unduly low alcohol concentration can lead to significant protein swelling, so that the free smaller fat globules can be incorporated in the protein. The percentage of fats bonded in the form of lipoproteins is not further considered here, since it may not interfere with liberation of the polar lipids (phospholipids).

In principle, it is believed that the higher the alcohol concentration, the stronger the protein contraction, but the more nonpolar the aqueous phase, more polar lipids may be dissolved in the oil phase. The appropriate concentration and temperature must therefore be found, for example, by conducting a few preliminary experiments (centrifuge tests), for each raw material.

Taking into consideration the natural moisture content of the raw material, aqueous alcohol is added to produce preferred final alcohol concentrations of 25-30%, and the dispersion is homogenized again. The contracted protein molecules and fat droplets are separated from each other. The intermediate layer between both, the polar lipid layer present on the surface of the fat globules, is thus disrupted. The oil therefore has an easier opportunity to be present as free phase in the dispersion. In order to reestablish equilibrium in this oil-in-water emulsion, on the one hand, the polar lipid could surround the fat globules again or, on the other hand, the oil droplets could coagulate to larger drops. For this purpose, the additional force of the centrifugal field is employed. The now large oil drops can then coalesce, i.e., forming a separable, continuous phase.

The procedure with a homogenizer is surprising for one skilled in the art as this produces very small oil droplets. In past methods, oil droplets were not reduced in size before being separated, because the degree of emulsion increases due to the larger internal surface area. Quite the contrary, agitation or kneading was carefully carried out, so that the oil can coagulate into larger drops. Heat was helpful in this malaxation process in order to also reduce the viscosity, among other things. The surprising effect that more oil can also be separated by a homogenization pressure increase to 300 bars or more may be explained by the interactions of the proteins, polar lipids and oil (actually, the nonpolar lipid phase) with the solvent layer.

Oil separation must therefore occur so that in general the surface tension and surface state of the droplets (destroyed as a result of shear) regain their original equilibrium. This means the homogenized slurry is preferably introduced immediately into a density separation device (preferably, a centrifuge of appropriate design and geometric considerations) and separated there into non-polar lipids (oil), and polar lipids with protein, water and alcohol. The viscosity reduction is not necessary to the degree it is necessary in oil recovery without homogenization (as described in WO 96/05278). Direct transfer of the homogenized slurry into the centrifugal field can be important in order to support coalescence.

After one- or two-stage oil separation, preferably in a decanter (other types of density separation devices, including centrifuges, are also successfully used for this purpose), all free oil fractions (lipids and nonpolar lipids) are ideally separated so that, by subsequent reduction of the alcohol concentration with water in the protein phase, no oil droplets are found in the free water/alcohol phase, although the polarity of the mixture is increased and lecithin is therefore bonded again in this phase and the oil therefore "liberated". Normally, the oil in this polar lipid/protein/alcohol mixture becomes free when the alcohol concentration is reduced; i.e., the oil solubility diminishes in the polar lipid phase. It was surprisingly found that, after two-fold homogenization and centrifuging, very little free oil was centrifugable, even if the alcohol concentration was only 15%.

Sterols including cholesterol may have a greater affinity for the polar lipid phase than for the oil phase, resulting in a higher sterol content in the polar lipid phase than in the oil phase. Movement of sterols into the oil or polar lipid phases can be manipulated by changing the pH of the mixture, altering temperature or by addition of processing aids such as salts to increase or decrease the polar nature of the aqueous phase. Another method to reduce the cholesterol in the polar lipid-rich fraction is to add oil with little or no cholesterol to the polar lipid-rich fraction and repeat the de-oiling process. In this way, the cholesterol can be segregated into the oil phase.

### EXAMPLE 1

Low Alcohol Extraction Process: One hundred kilograms of liquid egg yolk (containing 42 kg dry substance) was homogenized and then ethanol (35.4 kg of 96% purity) and 30.7 kg water were added to the egg yolk. The resulting alcohol concentration was 20% w/w overall (27% w/w referring only to alcohol and water). The mixture was then re-homogenized and the mixture was centrifuged using a decanter centrifuge yielding an oil phase and an alcohol/water phase. This de-oiling step yielded 17 kg egg yolk oil and 149 kg of the alcohol/water phase. The alcohol/water phase was then washed 3 times with the same low concentration of alcohol using a counter-current wash process employing a separator centrifuge. The process yielded two fractions: (1) a phospholipid-rich fraction (the liquid phase) which was dried to yield a product containing a total of 17 kg dry substance (containing 8 kg of phospholipid); and (2) a protein-rich fraction which was dried to yield 12 kg of dry substance (containing 11 kg or protein and 0.3 kg of phospholipid). Using an approximate average weight of 16.0 g per yolk, each containing about 1.7 g phospholipid per yolk, 100 kg egg yolk should yield approximately 10.6 kg of phospholipids. The 8.0 kg of phospholipids recovered in the phospholipid-rich fraction by this process represented a recovery efficiency for the phospholipid fraction of approximately 76%.

### EXAMPLE 2

Low Alcohol Extraction Process with High Alcohol Polishing Step: One hundred kilograms of liquid egg yolk (containing 42 kg dry substance) was homogenized and then ethanol and water were added to bring the mixture to a final alcohol concentration of 30% w/w in the alcohol/water phase. The mixture was then re-homogenized and the mixture was centrifuged using a decanter centrifuge yielding an oil phase and an alcohol/water phase. This de-oiling step yielded 16 kg egg yolk oil and 134 kg of the alcohol/water phase containing 26 kg dry substance. Seventy-two kg of ethanol and 170 kg water were then added to the alcohol/water phase, which was then mixed and centrifuged through a separator centrifuge. This yielded two fractions: (1) the liquid phase (299 kg) which contained 11 kg dry substance and (2) the solid phase (78 kg) which contained 15 kg dry substance. Fraction 1 contained the phospholipids with a small amount of proteins and Fraction 2 contained primarily proteins. Fraction I was then dried to a weight of 11.2 kg and 20 kg ethanol (96%) was added to this fraction. The mixture was then processed through a separator centrifuge yielding a liquid phase containing 10 kg dry substance. The liquid phase was then dried yielding a final weight of 10.5 kg (10.0.kg dry substance - the phospholipid fraction). The 78 kg solids in Fraction 2 were also dried resulting in 16 kg total (or 15 kg dry substance -the protein fraction). Using an approximate average weight of 16.0 g per yolk, each containing about 1.7 g phospholipid per yolk, 100 kg egg yolk should yield approximately 10.6 kg of phospholipids. The 10.0 kg of phospholipids recovered in this process represents a minimal recovery efficiency for the phospholipid fraction of greater than approximately 90%.

### EXAMPLE 3

Low Alcohol De-oiling Process with High Alcohol Polar Lipid Extraction Process: One hundred kilograms of liquid egg yolk (containing 45 kg dry substance) was homogenized and then ethanol and water were added to bring the mixture to a final alcohol concentration of 30% w/w in the alcohol/water phase. The mixture was then re-homogenized and the mixture was centrifuged using a decanter centrifuge yielding an oil phase and an alcohol/water phase. This de-oiling step yielded 17 kg egg yolk oil and 139 kg of the alcohol/water phase containing 28 kg dry substance. The alcohol/water phase was then dried (recovering 109 kg alcohol and water) yielding 30 kg material (containing 28 kg dry substance). Ninety kg ethanol (96% purity) was then added to this material and the mixture processed through a separator centrifuge yielding a liquid phase (containing the phospholipids) and a solid phase containing the proteins. The liquid phase (80 kg total containing 10.4 kg dry substance) was dried resulting in 10.6 kg of product containing 10.4 kg dry substance (phospholipids). The solid phase (40 kg total) was dried yielding 18.5 kg of product - protein (containing 17.6 kg of dry substance). Using an approximate average weight of 16.0 g per yolk, each containing about 1.7 g phospholipid per yolk, 100 kg egg yolk should yield approximately 10.6 kg of phospholipids. The 10.4 kg of phospholipids recovered in this process represents a minimum recovery efficiency for the phospholipid fraction of greater than approximately 90%.

The present invention, in various embodiments, includes components, methods, processes, systems and/or apparatus substantially as depicted and described herein, including various embodiments, subcombinations, and subsets thereof. Those of skill in the art will understand how to make and use the present invention after understanding the present disclosure. The present invention, in various embodiments, includes providing devices and processes in the absence of items not depicted and/or described herein or in various embodiments hereof, including in the absence of such items as may have been used in previous devices or processes, *e*.*g*., for improving performance, achieving ease and/or reducing cost of implementation.

## Claims

1. A process for fractionation of an oil-, polar lipid-, and protein-containing mixture, comprising the steps:
1.1) adding water-soluble organic solvent to said oil-, polar lipid-, and protein-containing mixture;
1.2) subjecting the oil-, polar lipid-, and protein-containing mixture to homogenisation; and
1.3) separating oil from said mixture to form an oil-rich fraction and a polar lipid/protein-rich fraction.

2. The process of claim 1, wherein said mixture is obtained from at least one of eggs, fish, crustaceans, microbes, brain tissue, milk, meat and plant material including oilseeds.

3. The process of any of claims 1 - 2, wherein said homogenisation is conducted at a pressure from 100x10⁵ Pa (100 bars) to 1000x10⁵ Pa (1000 bars).

4. The process of any of claims 1- 3, wherein said homogenisation is conducted at a pressure from 150x10⁵ Pa (150 bars) to 350x10⁵ Pa (350 bars).

5. The process as claimed in any of claims 1 - 4, wherein said oil-, polar lipid-, and protein-containing mixture is solubilised/dispersed in a water-soluble organic solvent and water mixture in which said water-soluble organic solvent comprises from 5% to 35% by weight of the total water-soluble organic solvent and water present.

6. The process as claimed in any of claims 1 - 5, wherein a density separation is performed to obtain the polar lipid/protein rich fraction the density separation being conducted in two steps, wherein in the first step the proportion of the water-soluble organic solvent in the total water-soluble organic solvent and water present amounts to 5% to 35% by weight and the density separation results in a first polar lipid/protein rich fraction, and wherein in the second step the proportion of the water-soluble organic solvent in the total water-soluble organic solvent and water present amounts to 68% to 98% by weight and the density separation results in a second polar lipid/protein rich fraction, wherein the second polar lipid/protein rich fraction contains a higher percentage of polar lipid than the first polar lipid/protein rich fraction.

7. A process for fractionation of an oil-, polar lipid-, and protein-containing mixture, comprising the steps:
a) separating oil from said mixture to form an oil-rich fraction and a polar lipid/protein-rich fraction;
b) adding water-soluble organic solvent to said polar lipid/protein-rich fraction; and
c) subjecting the water-soluble organic solvent and polar lipid/protein-rich fraction to density separation to form a polar lipid-rich fraction and a protein-rich fraction.
wherein the separation of oil of step a)
comprises the steps:
i. homogenisation of said oil-, polar lipid-, and protein-containing mixture,
ii. adding water-soluble organic solvent and water to said mixture; and
iii. separating the resulting mixture into an oil-rich fraction and a polar lipid/protein-rich fraction
or the steps of
iv. adding water-soluble organic solvent and water to said mixture;
v. homogenisation of said oil-, polar lipid-, and protein-containing mixture; and
vi. separating the resulting mixture into an oil-rich fraction and a polar lipid/protein-rich fraction.

8. The process of claim 7, wherein the separation of oil of step a) further comprises the step of homogenisation of the resulting mixture prior to separating the resulting mixture into an oil-rich fraction and a polar lipid/protein-rich fraction.

9. The process of any of claims 8 or 9, wherein said oil-, polar lipid-, and protein-containing mixture is derived from eggs.

10. The process of any of claims 8-9, wherein water-soluble organic solvent is recovered from the polar lipid-rich fraction and the protein-rich fraction after the density separation.

11. The process of any of claims 8 - 10, wherein said polar lipid/protein-rich fraction formed in step a) comprises from 30% to 50 % by weight polar lipid and from 50% to 70% by weight protein.

12. The process of claims 8-11, wherein said oil-rich fraction formed in step a) comprises from 75 % to 95 % by weight triacylglycerols.

13. The process of claims 8 - 12, wherein said oil-, polar lipid-, and protein-containing mixture further comprises cholesterol and a substantial amount of said cholesterol reports to said oil-rich fraction pursuant to the separation of step a).

14. The process of any of claims 8 -13, wherein said water-soluble organic solvent added in step b) forms a water-soluble organic solvent/water mixture in which said water-soluble organic solvent comprises from 20% to 35 % by weight of the total water-soluble organic solvent and water present.

15. The process of any of claims 8 - 17, wherein said water-soluble organic solvent added in step b) forms a water-soluble organic solvent/water mixture in which said water-soluble organic solvent comprises from 68% to 98% by weight of the total water-soluble organic solvent and water present.

16. The process of any of claims 8 - 15, wherein said water-soluble organic solvent is recovered by counter current washing, evaporation or drying.

17. The process of any of claims 8 - 15, wherein said polar lipid-rich fraction is dried to recover water-soluble organic solvent, washed with a water-soluble organic solvent/water mixture comprising greater than 80% by weight water-soluble organic solvent in order to precipitat rresdual protein and further dried to recover the water-soluble organic solvent.

18. The process of claim 17, wherein the addition of said water-soluble organic solvent results in the precipitation of at least some of said protein, which is recovered by density separation.

19. The process of any of claims 8 - 17, wherein residual protein is removed from said polar lipid-rich fraction by the addition of water.

20. The process of any of claims 8 - 19, wherein said water-soluble organic solvent comprises a polar solvent.

21. The process of any of claims 8 - 20, wherein said water-soluble organic solvent comprises an alcohol.

22. The process of any of claim 21, wherein said water-soluble organic solvent comprises a c1 - c8 alcohol.

23. The process of any of claims 21 - 22, wherein said water-soluble organic solvent comprises isopropanol, ethanol or mixtures thereof.

24. The process of any of claims 8 - 23, wherein the pH during processing is from pH 4 to pH 10.

25. The process of any of claims 8 - 24, wherein said mixture is obtained from at least one of eggs, fish, crustaceans, microbes, brain tissue, mild, meat and plant material including oilseeds.

26. The process of any of claims 8 - 25, wherein at least 60% of the polar lipids originally present in the mixture are recovered in a polar lipid-rich fraction.

27. The process of any of claims 8 - 26, wherein the temperature does not exceed 65 °C during the processing.

28. The process of any of claims 8 - 27, wherein said polar lipid comprises a phospholipid.

29. The process as claimed in any of claims 10-31, wherein the density separation is performed to obtain the polar lipid/protein rich fraction the density separation being conducted in two steps, wherein in the first step the proportion of the water-soluble organic solvent in the total water-soluble organic solvent and water present amounts to 5% to 35% by weight and the density separation results in a first polar lipid/protein rich fraction, and wherein in the second step the proportion of the water-soluble organic solvent in the total water-soluble organic solvent and water present amounts to 68% to 98% by weight and the density separation results in a second polar lipid/protein rich fraction, wherein the second polar lipid/protein rich fraction contains a higher percentage of polar lipid than the first polar lipid/protein rich fraction.

## Patentansprüche

1. Verfahren zur Fraktionierung eines Öl-, Polar-Lipid- und Protein-haltigen Gemisches, das folgende Schritte umfasst:
1.1) Zugabe eines wasserlöslichen, organischen Lösemittels zu dem Öl-, Polar-Lipid- und Protein-haltigen Gemisch;
1.2) Homogenisierung des Öl-, Polar-Lipid- und Protein-haltigen Gemischs; und
1.3) Trennen von Öl von dem Gemisch zur Ausbildung einer ölreichen Fraktion und einer Polar-Lipid-/Protein-reichen Fraktion.

2. Verfahren gemäß Anspruch 1, wobei das Gemisch von mindestens einem Element aus der Gruppe Eier, Fisch, Krustentiere, Mikroben, Gehirngewebe, Milch, Fleisch und Pflanzenmaterial einschließlich Ölsaaten gewonnen wird.

3. Verfahren gemäß einem der Ansprüche 1 -2, wobei die Homogenisierung bei einem Druck von 100x10⁵ Pa (100 Bar) bis 1000x10⁵ Pa (1000 Bar) durchgeführt wird.

4. Verfahren gemäß einem der Ansprüche 1 - 3, wobei die Homogenisierung bei einem Druck von 150x10⁵ Pa (150 Bar) bis 350x10⁵ Pa (350 Bar) durchgeführt wird.

5. Verfahren gemäß einem der Ansprüche 1 - 4, wobei das Öl-, Polar-Lipid- und Protein-haltige Gemisch in einem Gemisch aus einem wasserlöslichen, organischen Lösemittel und Wasser gelöst/dispergiert wird, in dem das wasserlösliche, organische Lösemittel von 5 bis 35 Gewichtsprozent des gesamten vorhandenen Gemischs aus wasserlöslichem, organischem Lösemittel und Wasser ausmacht.

6. Verfahren gemäß einem der Ansprüche 1 - 5, wobei eine Dichtetrennung durchgeführt wird, um die Polar-Lipid-/Protein-reiche Fraktion zu gewinnen, wobei die Dichtetrennung in zwei Schritten durchgeführt wird, wobei im ersten Schritt der Anteil des wasserlöslichen, organischen Lösemittels am gesamten vorhandenen Gemisch aus wasserlöslichem, organischem Lösemittel und Wasser von 5 bis 35 Gewichtsprozent ausmacht und die Dichtetrennung eine erste Polar-Lipid-/Protein-reiche Fraktion ergibt, und wobei im zweiten Schritt der Anteil des wasserlöslichen, organischen Lösemittels am gesamten vorhandenen Gemisch aus wasserlöslichem, organischem Lösemittel und Wasser von 68 bis 98 Gewichtsprozent ausmacht und die Dichtetrennung eine zweite Polar-Lipid-/Protein-reiche Fraktion ergibt, wobei die zweite Polar-Lipid-/Protein-reiche Fraktion einen höheren Anteil an polarem Lipid enthält als die erste Polar-Lipid-/Protein-reiche Fraktion.

7. Verfahren zur Fraktionierung eines Öl-, Polar-Lipid- und Protein-haltigen Gemischs, das folgende Schritte umfasst:
a) Trennen des Öls von dem Gemisch zur Ausbildung einer Öl-reichen Fraktion und einer Polar-Lipid-/Protein-reichen Fraktion;
b) Zugabe eines wasserlöslichen, organischen Lösemittels zu der Polar-Lipid-/Protein-reichen Fraktion; und
c) Dichtetrennung des wasserlöslichen, organischen und der Polar-Lipid-/Protein-reichen Fraktion zur Ausbildung einer Polar-Lipid-reichen Fraktion und einer Protein-reichen Fraktion,
wobei die Trennung des Öls in Schritt a) folgende Schritte umfasst:
i. Homogenisierung des Öl-, Polar-Lipid- und Protein-haltigen Gemischs,
ii. Zugabe eines wasserlöslichen, organischen Lösemittels und Wassers zu dem Gemisch; und
iii. Trennen des resultierenden Gemischs in eine Öl-reiche Fraktion und eine Polar-Lipid-/Protein-reiche Fraktion,
oder folgende Schritte umfasst:
iv. Zugabe eines wasserlöslichen, organischen Lösemittels und Wassers zu dem Gemisch;
v. Homogenisierung des Öl-, Polar-Lipid- und Protein-haltigen Gemischs; und
vi. Trennen des resultierenden Gemischs in eine Öl-reiche Fraktion und eine Polar-Lipid-/Protein-reiche Fraktion.

8. Verfahren gemäß Anspruch 7, wobei die Trennung des Öls in Schritt a) ferner den Schritt der Homogenisierung des resultierenden Gemischs vor der Trennung des resultierenden Gemischs in eine Öl-reiche Fraktion und eine Polar-Lipid-reiche Fraktion umfasst.

9. Verfahren gemäß Anspruch 8 oder 9, wobei das Öl-, Polar-Lipid- und Protein-haltige Gemisch aus Eiern gewonnen wird.

10. Verfahren gemäß einem der Ansprüche 8 - 9, wobei das wasserlösliche, organische Lösemittel aus der Polar-Lipid-reichen Fraktion und der Protein-reichen Fraktion nach der Dichtetrennung gewonnen wird.

11. Verfahren gemäß einem der Ansprüche 8 - 10, wobei die in Schritt a) ausgebildete Polar-Lipid-/Protein-reiche Fraktion von 30 bis 50 Gewichtsprozent polares Lipid und von 50 bis 70 Gewichtsprozent Protein umfasst.

12. Verfahren der Ansprüche 8 - 11, wobei die in Schritt a) ausgebildete Öl-reiche Fraktion von 75 bis 95 Gewichtsprozent Triacylglycerole umfasst.

13. Verfahren der Ansprüche 8 - 12, wobei das Öl-, Polar-Lipid- und Protein-haltige Gemisch ferner Cholesterin umfasst und eine substanzielle Menge des Cholesterins der Öl-reichen Fraktion gemäß der Trennung des Schritts a) reportiert.

14. Verfahren gemäß einem der Ansprüche 8 - 13, wobei das in Schritt b) hinzugefügte wasserlösliche, organische Lösemittel ein Gemisch aus einem wasserlöslichen, organischen Lösemittel und Wasser bildet, in dem das wasserlösliche, organische Lösemittel von 20 bis 35 Gewichtsprozent des gesamten vorhandenen wasserlöslichen, organischen Lösemittels umfasst.

15. Verfahren gemäß einem der Ansprüche 8 - 17, wobei das in Schritt b) hinzugefügte wasserlösliche, organische Lösemittel ein Gemisch aus wasserlöslichem, organischem Lösemittel und Wasser bildet, in dem das wasserlösliche, organische Lösemittel von 68 bis 98 Gewichtsprozent des gesamten vorhandenen wasserlöslichen, organischen Lösemittels und Wassers umfasst.

16. Verfahren gemäß einem der Ansprüche 8 - 15, wobei das wasserlösliche, organische Lösemittel durch Gegenstromwaschen, Verdampfen oder Trocknen gewonnen wird.

17. Verfahren gemäß einem der Ansprüche 8 - 16, wobei die Polar-Lipid-reiche Fraktion getrocknet wird, um wasserlösliches, organisches Lösemittel zu gewinnen, mit einem Gemisch aus wasserlöslichem, organischem Lösemittel und Wasser, das mehr als 80 Gewichtsprozent wasserlösliches, organisches Lösemittel enthält, gewaschen wird, um restliches Protein auszufällen, und ferner getrocknet wird, um das wasserlösliche, organische Lösemittel zu gewinnen.

18. Verfahren gemäß Anspruch 17, wobei die Zugabe des wasserlöslichen, organischen Lösemittels die Ausfällung zumindest eines Teils des Proteins bewirkt, das durch die Dichtetrennung gewonnen wird.

19. Verfahren gemäß einem der Ansprüche 8 - 17, wobei das restliche Protein von der Polar-Lipid-reichen Fraktion durch die Zugabe von Wasser entfernt wird.

20. Verfahren gemäß einem der Ansprüche 8 - 19, wobei das wasserlösliche, organische Lösemittel ein polares Lösemittel umfasst.

21. Verfahren gemäß einem der Ansprüche 8 - 20, wobei das wasserlösliche, organische Lösemittel einen Alkohol enthält.

22. Verfahren gemäß Anspruch 21, wobei das wasserlösliche, organische Lösemittel einen c1 - c8 Alkohol enthält.

23. Verfahren gemäß einem der Ansprüche 21- 22, wobei das wasserlösliche, organische Lösemittel Isopropanol, Ethanol oder Gemische daraus umfasst.

24. Verfahren gemäß einem der Ansprüche 8 - 23, wobei der pH-Wert während der Verarbeitung von pH 4 bis pH 10 reicht.

25. Verfahren gemäß einem der Ansprüche 8 - 24, wobei das Gemisch aus mindestens einem Element aus der Gruppe Eier, Fisch, Krustentiere, Mikroben, Gehirngewebe, Milch, Fleisch und Pflanzenmaterial einschließlich Ölsaaten gewonnen wird.

26. Verfahren gemäß einem der Ansprüche 8 - 25, wobei mindestens 60% der polaren Lipide, die ursprünglich im Gemisch vorhanden sind, in einer Polar-Lipid-reichen Fraktion gewonnen werden.

27. Verfahren gemäß einem der Ansprüche 8 - 26, wobei die Temperatur während der Verarbeitung 65°C nicht übersteigt.

28. Verfahren gemäß einem der Ansprüche 8 - 27, wobei das polare Lipid ein Phospholipid umfasst.

29. Verfahren gemäß einem der Ansprüche 10 - 31, wobei die Dichtetrennung durchgeführt wird, um die Polar-Lipid-/Protein-reiche Fraktion zu gewinnen, wobei die Dichtetrennung in zwei Schritten durchgeführt wird, wobei im ersten Schritt der Anteil des wasserlöslichen, organischen Lösemittels am gesamten vorhandenen Gemisch aus wasserlöslichem, organischem Lösemittel und Wasser von 5 bis 35 Gewichtsprozent ausmacht und die Dichtetrennung eine erste Polar-Lipid-/Protein-reiche Fraktion ergibt, und wobei im zweiten Schritt der Anteil des wasserlöslichen, organischen Lösemittels am gesamten vorhandenen Gemisch aus wasserlöslichem, organischem Lösemittel und Wasser von 68 bis 98 Gewichtsprozent ausmacht und die Dichtetrennung eine zweite Polar-Lipid-/Protein-reiche Fraktion ergibt, wobei die zweite Polar-Lipid-/Protein-reiche Fraktion einen höheren Anteil an polarem Lipid enthält als die erste Polar-Lipid-/Protein-reiche Fraktion.

## Revendications

1. Procédé de fractionnement d'un mélange contenant de l'huile, des lipides polaires et des protéines, comprenant les étapes consistant à :
1.1) ajouter du solvant organique soluble dans l'eau audit mélange contenant de l'huile, des lipides polaires et des protéines ;
1.2) soumettre le mélange contenant de l'huile, des lipides polaires et des protéines à homogénéisation ; et
1.3) séparer l'huile dudit mélange pour former une fraction riche en huile et une fraction riche en lipides polaires/protéines.

2. Procédé selon la revendication 1, dans lequel ledit mélange est obtenu à partir d'au moins un parmi des oeufs, du poisson, des crustacées, des microbes, du tissu cérébral, du lait, de la viande et d'un végétal comprenant les graines oléagineuses.

3. Procédé selon l'une quelconque des revendications 1 ou 2, dans lequel ladite homogénéisation est opérée à une pression de 100 x 10⁵ Pa (100 bars) à 1 000 x 10⁵ Pa (1 000 bars).

4. Procédé selon l'une quelconque des revendications 1 à 3, dans lequel ladite homogénéisation est opérée à une pression de 150 x 10⁵ Pa (150 bars) à 350 x 10⁵ Pa (350 bars).

5. Procédé selon l'une quelconque des revendications 1 à 4, dans lequel ledit mélange contenant de l'huile, des lipides polaires et des protéines est solubilisé/dispersé dans un mélange de solvant organique soluble dans l'eau et d'eau dans lequel ledit solvant organique soluble dans l'eau représente 5 % à 35 % en poids du total du solvant organique soluble dans l'eau et de l'eau présents.

6. Procédé selon l'une quelconque des revendications 1 à 5, dans lequel une séparation par densité est effectuée pour obtenir la fraction riche en lipides polaires/protéines, la séparation par densité étant effectuée en deux étapes, dans lequel dans la première étape, la proportion du solvant organique soluble dans l'eau dans le total du solvant organique soluble dans l'eau et de l'eau présents atteint 5 % à 35 % en poids et la séparation par densité aboutit à une première fraction riche en lipides polaires/protéines, et dans lequel dans la seconde étape, la proportion du solvant organique soluble dans l'eau dans le total du solvant organique soluble dans l'eau et de l'eau présents atteint 68 % à 98 % en poids et la séparation par densité aboutit à une seconde fraction riche en lipides polaires/protéines, dans lequel la seconde fraction riche en lipides polaires/protéines contient un pourcentage plus élevé de lipides polaires que la première fraction riche en lipides polaires/protéines.

7. Procédé de fractionnement d'un mélange contenant de l'huile, des lipides polaires et des protéines, comprenant les étapes consistant à :
a) séparer l'huile dudit mélange pour former une fraction riche en huile et une fraction riche en lipides polaires/protéines ;
b) ajouter un solvant organique soluble dans l'eau à ladite fraction riche en lipides polaires/protéines ; et
c) soumettre le solvant organique soluble dans l'eau et la fraction riche en lipides polaires/protéines à la séparation par densité pour former une fraction riche en lipides polaires et une fraction riche en protéines,
dans lequel la séparation d'huile de l'étape a) comprend les étapes consistant à :
i. homogénéiser ledit mélange contenant de l'huile, des lipides polaires et des protéines,
ii. ajouter un solvant organique soluble dans l'eau et de l'eau audit mélange ; et
iii. séparer le mélange obtenu en une fraction riche en huile et une fraction riche en lipides polaires/protéines
ou les étapes consistant à
iv. ajouter un solvant organique soluble dans l'eau et de l'eau audit mélange ;
v. homogénéiser ledit mélange contenant de l'huile, des lipides polaires et des protéines ; et
vi. séparer le mélange obtenu en une fraction riche en huile et une fraction riche en lipides polaires/protéines.

8. Procédé selon la revendication 7, dans lequel la séparation d'huile de l'étape a) comprend en outre l'étape consistant à homogénéiser le mélange obtenu avant de séparer le mélange obtenu en une fraction riche en huile et une fraction riche en lipides polaires/protéines.

9. Procédé selon l'une quelconque des revendications 8 ou 9, dans lequel ledit mélange contenant de l'huile, des lipides polaires et des protéines est dérivé des oeufs.

10. Procédé selon l'une quelconque des revendications 8 ou 9, dans lequel le solvant organique soluble dans l'eau est récupéré à partir de la fraction riche en lipides polaires et de la fraction riche en protéines après la séparation par densité.

11. Procédé selon l'une quelconque des revendications 8 à 10, dans lequel ladite fraction riche en lipides polaires/protéines formée dans l'étape a) comprend 30 % à 50 % en poids de lipides polaires et 50 % à 70 % en poids de protéines.

12. Procédé selon les revendications 8 à 11, dans lequel ladite fraction riche en huile formée dans l'étape a) comprend 75 % à 95 % en poids de triacylglycérols.

13. Procédé selon les revendications 8 à 12, dans lequel ledit mélange contenant de l'huile, des lipides polaires et des protéines comprend en outre du cholestérol et une quantité substantielle dudit cholestérol se rapporte à ladite fraction riche en huile suite à la séparation de l'étape a).

14. Procédé selon l'une quelconque des revendications 8 à 13, dans lequel ledit solvant organique soluble dans l'eau ajouté dans l'étape b) forme un mélange de solvant organique soluble dans l'eau et d'eau dans lequel ledit solvant organique soluble dans l'eau représente 20 % à 35 % en poids du total du solvant organique soluble dans l'eau et de l'eau présents.

15. Procédé selon l'une quelconque des revendications 8 à 17, dans lequel ledit solvant organique soluble dans l'eau ajouté dans l'étape b) forme un mélange de solvant organique soluble dans l'eau et d'eau dans lequel ledit solvant organique soluble dans l'eau représente 68 % à 98 % en poids du total du solvant organique soluble dans l'eau et de l'eau présents.

16. Procédé selon l'une quelconque des revendications 8 à 15, dans lequel ledit solvant organique soluble dans l'eau est récupéré par lavage à contre-courant, évaporation ou séchage.

17. Procédé selon l'une quelconque des revendications 8 à 16, dans lequel ladite fraction riche en lipides polaires est séchée pour récupérer le solvant organique soluble dans l'eau, lavée avec un mélange de solvant organique soluble dans l'eau et d'eau comprenant plus de 80 % en poids de solvant organique soluble dans l'eau afin de précipiter les protéines résiduelles et séchée ensuite pour récupérer le solvant organique soluble dans l'eau.

18. Procédé selon la revendication 17, dans lequel l'ajout dudit solvant organique soluble dans l'eau entraîne la précipitation d'au moins une partie de ladite protéine, qui est récupérée par séparation par densité.

19. Procédé selon l'une quelconque des revendications 8 à 17, dans lequel une protéine résiduelle est éliminée de ladite fraction riche en lipides polaires par l'ajout d'eau.

20. Procédé selon l'une quelconque des revendications 8 à 19, dans lequel ledit solvant organique soluble dans l'eau comprend un solvant polaire.

21. Procédé selon l'une quelconque des revendications 8 à 20, dans lequel ledit solvant organique soluble dans l'eau comprend un alcool.

22. Procédé selon la revendication 21, dans lequel ledit solvant organique soluble dans l'eau comprend un alcool en c1-c8.

23. Procédé selon l'une quelconque des revendications 21 ou 22, dans lequel ledit solvant organique soluble dans l'eau comprend de l'isopropanol, de l'éthanol ou des mélanges de ceux-ci.

24. Procédé selon l'une quelconque des revendications 8 à 23, dans lequel le pH pendant le traitement est de pH 4 à pH 10.

25. Procédé selon l'une quelconque des revendications 8 à 24, dans lequel ledit mélange est obtenu à partir d'au moins un parmi des oeufs, du poisson, des crustacés, des microbes, du tissu cérébral, du lait, de la viande et de végétaux comprenant les graines oléagineuses.

26. Procédé selon l'une quelconque des revendications 8 à 25, dans lequel au moins 60 % des lipides polaires présents à l'origine dans le mélange sont récupérés dans une fraction riche en lipides polaires.

27. Procédé selon l'une quelconque des revendications 8 à 26, dans lequel la température ne dépasse pas 65 °C pendant le traitement.

28. Procédé selon l'une quelconque des revendications 8 à 27, dans lequel ledit lipide polaire comprend un phospholipide.

29. Procédé selon l'une quelconque des revendications 10 à 31, dans lequel la séparation par densité est effectuée pour obtenir la fraction riche en lipides polaires/protéines, la séparation par densité étant effectuée en deux étapes, dans lequel dans la première étape, la proportion du solvant organique soluble dans l'eau dans le total du solvant organique soluble dans l'eau et de l'eau présents atteint 5 % à 35 % en poids et la séparation par densité aboutit à une première fraction riche en lipides polaires/protéines, et dans lequel dans la seconde étape, la proportion du solvant organique soluble dans l'eau dans le total du solvant organique soluble dans l'eau et de l'eau présents atteint 68 % à 98 % en poids et la séparation par densité aboutit à une seconde fraction riche en lipides polaires/protéines, dans lequel la seconde fraction riche en lipides polaires/protéines contient un pourcentage plus élevé de lipides polaires que la première fraction riche en lipides polaires/protéines.
